# EUROPÄISCHE PATENTANMELDUNG

(11) **EP 2 730 309 A1**
(43) Veröffentlichungstag der Anmeldung: **14.05.2014**
(21) Anmeldenummer: 13191236.2
(22) Anmeldetag: 01.11.2013
(51) Int. Cl.: A61M 25/10

(54) **Ballonkatheter für gekrümmte Gefäße**

(30) Priorität: 13.11.2012 US 201261725492 P
(71) Anmelder: Biotronik AG, 8180 Bülach (CH)
(72) Erfinder: Pöhlmann, Stefanie, 95466 Weidenberg (DE); Amido, Alexandre, 18057 Rostock (DE); Wesselmann, Matthias, 8455 Rüdlingen (CH)
(74) Vertreter: Galander, Marcus

(57) **Zusammenfassung**

Die Erfindung betrifft einen Ballonkatheter zur Behandlung einer Stenose in einem Körpergefäß 2, insbesondere zum Einbringen eines Stents 1 in das Körpergefäß 2, wobei der Ballon 10 derart ausgebildet ist, dass er im expandierten Zustand die gekrümmte oder gebogene dreidimensionale Form des Körpergefäßes 2 annimmt.

## Beschreibung

Die vorliegende Erfindung betrifft einen Ballonkatheter mit einem expandierbaren Ballon zur Behandlung von Stenosen in Körpergefäßen. Ferner betrifft die Erfindung ein System zum Einbringen eines Stents in ein Körpergefäß.

Unter einer Stenose eines Körpergefäßes wird die Verengung des Gefäßdurchmessers verstanden. In Körpergefäßen insbesondere in Arterien, Venen oder ähnlichen Hohlorganen kann es zu Verengungen durch Ablagerungen kommen. Dadurch wird der Fluss durch diese Gefäße eingeschränkt und im schlimmsten Fall unterbunden. Die Körpergefäße werden nicht mehr von den Körperflüssigkeiten durchströmt und stromabwärts gelegenen Regionen nicht mehr bzw. nicht ausreichend versorgt.

Derartige Stenosen in Arterien oder Venen können medizinisch durch ein Angioplastie genanntes Verfahren behandelt werden. Bei einer Angioplastie werden die verengten Gefäße mechanisch wieder aufgeweitet, wodurch der durchströmbare Durchmesser der Körpergefäße wieder erhöht wird. Dabei wird ein Ballonkatheter mit einem expandierbaren Ballon in das Körpergefäß derart geführt, dass sich der expandierbare Ballon an der Stelle der Stenose, d.h. der Verengung, befindet. Durch die Expansion des Ballons wird das Körpergefäß wieder aufgeweitet. Die Gefäßwände werden aufgeweitet, die Ablagerung an die Gefäßwand gedrückt.

Um die dauerhafte Aufweitung zu gewährleisten, wird dabei häufig eine Gefäßstütze, ein so genannter Stent, in das Köpergefäß an die betroffene Stelle eingebracht. Häufig weisen dabei auch Ballon und/oder Stent eine Beschichtung auf, die über einen vorgegebenen Zeitraum medizinisch wirksame Substanzen abgibt, um die Heilung zu verbessern und/oder die Ausbildung einer erneuten Verengung (Restenose) zu verhindern.

Unter der Behandlung einer Stenose in einem Körpergefäß wird daher im Rahmen dieser Anmeldung, die Behandlung einer Verengung in einem Körpergefäß, insbesondere in einer Arterie oder Vene, verstanden, wobei ein Ballonkatheter in das betroffene Körpergefäß eingebracht wird. Die Behandlung umfasst dabei das Aufweiten der Verengung durch einen expandierbaren Ballon des Ballonkatheters mit oder ohne Einbringen eines Stents mit oder ohne Beschichtung des Ballons und/oder Stents.

Unter einem Stent wird im Rahmen der Anmeldung ein Metall oder Polymergeflecht verstanden, welches in das Körpergefäß eingebracht werden kann. Der Stent bewirkt dabei zumindest über eine vorgegebene Zeit eine Stützung des Körpergefäßes. Der Stent kann im Rahmen dieser Anmeldung sowohl permanent als auch biologisch abbaubar oder degradierbar sein.

Im Stand der Technik sind verschiedene Ballonkatheter mit expandierbaren Ballon bekannt. Im Rahmen dieser Anmeldung werden die Begriffe Expansion/expandieren, Dilatation/dilatieren und Aufdehnung/aufdehnen des Ballons als synonym betrachtet, wobei im Rahmen dieser Anmeldung der Begriff Expansion/expandieren verwendet wird.

Die im Stand der Technik bekannten Ballonkatheter weisen alle die folgenden wesentlichen Bestandteile auf: einen Ballon, eine Spitze, ein inneres Lumen für einen Führungsdraht und ein Lumen zur Beaufschlagung des Ballons mit einem Fluid sowie die entsprechenden Verbindungsmöglichkeiten mit einer Druckquelle für das Fluid.

Unter einem Lumen wird im Rahmen dieser Anmeldung der freie durchströmbare Querschnitt eines Hohlkörpers verstanden. Im einfachsten Fall eines zylindrischen Rohrs ergibt sich das Lumen entsprechend als innerer Querschnitt des Rohres.

Ein Ballonkatheter weist einen Schaft auf, in dessen distalen Bereich sich der expandierbare Ballon befindet. Im proximalen Bereich bzw. am proximalen Ende des Ballonkatheters befinden sich die Verbindungsmöglichkeiten (zumeist ein oder mehrere Luer Lock Anschlüsse) für die Zuführung des Führungsdrahtes bzw. für das Fluid zur Beaufschlagung des Ballons.

Im Rahmen dieser Anmeldung wird unter dem proximalen Ende eines Ballonkatheters das Ende verstanden, welches in der Hand des Behandlers außerhalb des Körpers/des Körpergefäßes liegt. Das distale Ende eines Ballonkatheters ist entsprechend die in das Körpergefäß geführte Spitze des Ballonkatheters. Entsprechend sind die Lagebezeichnungen proximal (näher zum Behandler) und distal (näher zur im Körpergefäß befindlichen Spitze des Ballonkatheters) zu verstehen.

Zumeist wurde ein Führungsdraht, welcher mit Röntgenmarkern ausgestattet oder komplett sichtbar für Röntgenstrahlung ist, schon im Körpergefäß platziert. Über diesen Führungsdraht wird der Ballonkatheter mit der Spitze voran eingeführt. Die Spitze des Ballonkatheters ist dabei sehr flexibel, um Verletzungen der Wände des Körpergefäßes zu vermeiden, und derart beschichtet, dass ein möglichst reibungsfreies Gleiten im Körpergefäß ermöglicht wird. Zusätzlich ist sie sichtbar bei Röntgenstrahlung. Der Führungsdraht wird im inneren Lumen des Ballonkatheters geführt.

Der Ballonkatheter wird mit Hilfe des Führungsdrahtes so platziert, dass der Ballon an der Stelle der Stenose im Gefäß ist. Um eine sichere Platzierung zu gewährleisten, weißt der Schaft und/oder der Ballon entsprechende Röntgenmarkierungen auf. Dann wird der Ballon über das entsprechende Lumen mit einem Fluid beaufschlagt und expandiert. Die Ablagerungen werden dabei an die Gefäßwand gedrückt und das Gefäß erweitert. Der Ballon wird dabei mit einem Druck von 6 bar (4 bis 8 bar, Niederdruckexpansion) oder bei einer Hochdruckexpansion mit einem Druck von mehr als 16 bis 18 bar bzw. bis zu 40 bar beaufschlagt.

Je nach Anordnung der Lumen des Ballonkatheters wird dabei im Stand der Technik zwischen einem Over-the-Wire System, einem Rapid-Exchange System oder einem Fixed-Wire System unterschieden. Bei einem Over-the-Wire System sind das innere Lumen für den Führungsdraht und das Lumen zur Fluidbeaufschlagung des Ballons koaxial angeordnet. Im inneren Schaft mit dem inneren Lumen befindet sich der Führungsdraht, der äußere Schaft mit größeren Innen- und Außendurchmesser bildet das Lumen zur Beaufschlagung des Ballons. Beide Lumen erstrecken sich vom proximalen bis zum distalen Ende des Ballonkatheters. Bei einem Rapid-Exchange System (teilweise auch als Monorail System oder Single-Operator System bekannt) ist das Lumen für den Führungsdraht nicht bis zum proximalen Ende des Ballonkatheters durchgehend, sondern nur im distalen Abschnitt des Ballonkatheters (rund 20cm). Dies erlaubt einen schnellen Austausch des Ballonkatheters und die Verwendung kürzerer Führungsdrähte. Das Lumen für den Führungsdraht und das Lumen zur Fluidbeaufschlagung des Ballons können dabei koaxial oder parallel angeordnet sein. Fixed-Wire Ballonkatheter haben einen Führungsdraht, der in ihrem Lumen fest positioniert ist.

Im Rahmen dieser Anmeldung wird das Lumen für den Führungsdraht immer als inneres Lumen (und entsprechend innerer Schaft) und das Lumen zur Fluidbeaufschlagung des Ballons immer als äußeres Lumen bezeichnet, unabhängig von koaxialer oder paralleler Anordnung. Das Innere, mit Fluid befüllbar und unter Druck setzbare, des Ballons wird im Rahmen dieser Anmeldung als Ballonlumen oder Balloninnere bezeichnet.

Werden derartige Ballonkatheter nach dem Stand der Technik zur Implantierung von Stents in Körpergefäßen verwendet, wird auf den expandierbaren Ballon ein Stent aufgebracht. Dies wird im Stand der Technik auch als crimpen bezeichnet. Mit der Expansion des Ballons wird dann der Stent aufgedehnt und gegen die Gefäßwand gepresst. Der Stent ist dabei aus einem derartigen Material, dass er nach der Expansion die dabei erhaltene Form behält.

Im Stand der Technik sind unterschiedliche Ballone und Stents in den verschiedensten Größen bekannt. Als Standardgröße wird im Stand der Technik beispielsweise ein Ballon verstanden, der für die koronare Anwendung einen Durchmesser von 3.0 mm im expandierten Zustand und eine Länge von 20 mm aufweist. Der zugehörige Stent weist in seinem expandierten Zustand analoge Dimensionen auf.

Der nicht expandierte Ballon wird auf den inneren Schaft gefaltet, der Stent gegebenenfalls darauf gepresst, so dass das sich im nicht expandierten Zustand eine möglichst kleiner

Durchmesser ergibt, um den Ballonkatheter möglichst einfach und störungsfrei in das Körpergefäß an die Stelle der Stenose zu führen. Bei Expansion nehmen sowohl der Ballon als auch gegebenenfalls der darauf befestigte Stent eine im Wesentlichen zylindrische Form an.

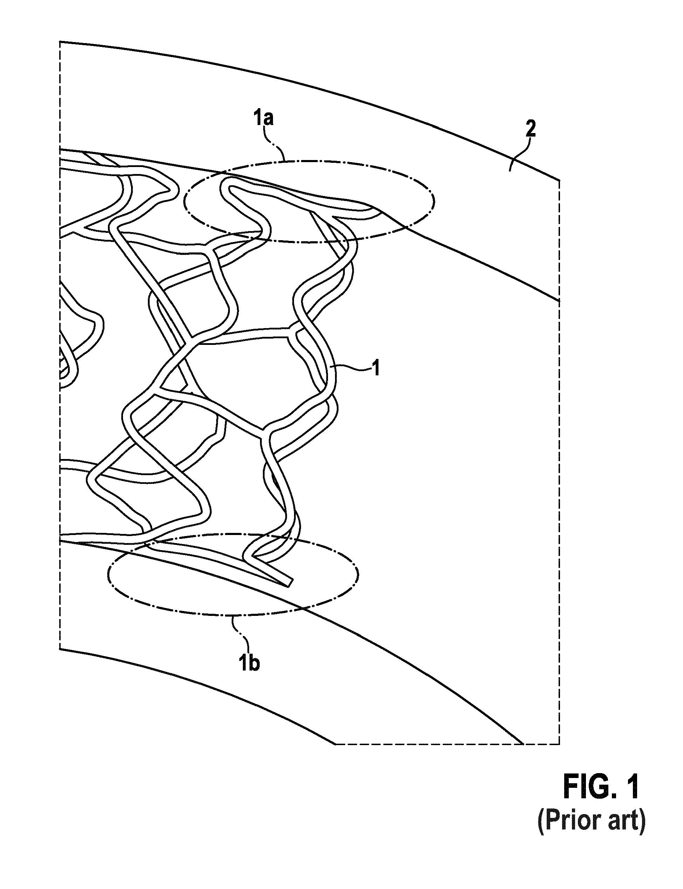

Dies führt bei gekrümmten Körpergefäßen zu Problemen, insbesondere wenn die Stenose an einer Krümmung des Körpergefäßes auftritt. In diesem Fall führt die Expansion des Ballons zu einer unerwünschten und unphysiologischen Begradigung des Körpergefäßes bedingt durch die hohe Biegesteifigkeit des inflatierten Ballons, die insbesondere bei Einsatz eines permanenten Stents erhalten bleibt. Wird ein Stent in ein derart gekrümmtes Gefäß eingebracht, wird der Teil des Gefäßes, der unmittelbar vom Stent gestützt wird, begradigt. Die benachbarten Gefäßabschnitte behalten im Wesentlichen ihre vorherige Form, so dass es an den Rändern des Stents zu einem Abknicken des Körpergefäßes kommt, wie in Figur 1 dargestellt.

Figur 1 zeigt einen Stent 1 in ein Körpergefäß 2 nach Implantation mit einem Ballonkatheter nach dem vorangehend geschilderten Stand der Technik. Durch die unphysiologische Begradigung im Bereich des implantierten Stents 1 ist das Gefäß 2 am Rand des Stents 1 abgeknickt. Dabei drückt der Stent 1 im Außenbereich 1a des abgeknickten Gefäßes 2 in die Gefäßwand, wodurch dort starke Spannungen in das Körpergefäß 2 eingebracht werden. Dies führt nach neueren Untersuchungen zur Erhöhung des Restenoserisikos. Im Innenbereich 1i des abgeknickten Gefäßes liegt der Stent 1 nicht gut an der Wand des Gefäßes 2 an. Dadurch bildet sich dort ein Bereich mit einer deutlich reduzierten Strömung, wodurch ebenfalls das Risiko einer Restenose steigt.

Der vorliegenden Erfindung liegt daher die Aufgabe zugrunde, einen Ballonkatheter der vorangehend geschilderten Art, insbesondere einen Ballonkatheter zum Einbringen eines Stents in ein Körpergefäß, derart auszugestalten, dass der Ballon im expandierten Zustand den natürlichen Verlauf des Körpergefäßes möglichst gut nachbildet. Ein Begradigen des Körpergefäßes durch den expandierten Ballon, insbesondere durch einen Stent, der mittels expandierten Ballons an die Gefäßwand gepresst wird, soll bei der vorliegenden Erfindung vermieden werden.

Die vorliegende Aufgabe wird durch die Merkmale des unabhängigen Anspruches 1 gelöst. Weitere vorteilhafte Ausgestaltungen werden in den abhängigen Ansprüchen aufgeführt.

Erfindungsgemäß wird der Ballon derart ausgeformt ist, dass er im expandierten Zustand eine Form einnimmt, die von der Form eines Zylinders abweicht, insbesondere der Ballon im expandierten Zustand eine gebogene oder gewundene dreidimensionale Form aufweist. Hauptsächlich erfolgt die Begradigung der gekrümmten Gefäße durch die im Wesentlichen zylindrische Form der expandierten Ballone eines Ballonkatheters nach dem Stand der Technik. Dies wird durch die vorliegende Erfindung vermieden. Die Form des expandierten Ballons bei einem erfindungsgemäßen Ballonkatheter ist nicht zylindrisch. Dadurch werden die Begradigung und die damit einhergehenden negativen Effekte durch die Expansion des Ballons, und die optional damit verbundene Implantation des Stents, vermieden.

Unter expandierten Zustand des Ballons wird im Rahmen der Anmeldung verstanden, dass der Ballon an seiner bestimmungsgemäßen Position im Körpergefäß mit einem Fluid beaufschlagt und expandiert wurde. Dies umfasst sowohl die vorangehend beschriebenen Niederdruckexpansion als auch die Hochdruckexpansion.

Vorteilhafterweise ist der Ballon derart ausgeformt, dass er im expandierten Zustand der Form des umgebenden Körpergefäßes angepasst ist, insbesondere eine gebogene oder gewundene dreidimensionale Form aufweist. Die vorausgehend geschilderten Probleme treten bei Körpergefäßen auf, deren Form von einer geraden zylindrischen Form abweicht, d.h. sich die Stenose an einer Stelle gebildet hat, wo das Körpergefäß gebogen ist. Durch den erfindungsgemäßen Ballonkatheter kann hier vorteilhafterweise der Ballon expandiert und damit das Körpergefäße aufgedehnt werden, ohne dass das Körpergefäß begradigt wird. Der Ballon hat in seinem expandierten Zustand eine gebogene oder gewundene dreidimensionale Form, die der Form des zu behandelnden Körpergefäßes entspricht. Dadurch wird vorteilhafterweise auch bei Einbringen eines Stents in das Körpergefäß der Stent auf eine gebogene oder gewundene dreidimensionale Form aufgedehnt. Die aufgedehnte Form des Stents entspricht der Form des Körpergefäßes, so dass der Stent optimal dem Körpergefäß angepasst ist, das Körpergefäß durch den eingebrachten Stent nicht begradigt wird, sondern in seiner natürlichen dreidimensionalen Form bleibt. Dadurch werden die vorausgehend geschilderten Effekte des Standes der Technik am Innenradius (Strömungstotstellen) und am Außenradius (starke Spannungen in der Gefäßwand durch den Stent) vermieden und insgesamt das Risiko einer Restenose gesenkt.

Gemäß einer vorteilhaften Ausgestaltung der Erfindung ist der Ballon aus mindestens zwei, bevorzugt drei, einzelnen Segmenten zusammengesetzt, wobei mindestens ein Segment eine Fläche aufweist, die kein Rechteck ist. Gemäß dieser Ausgestaltung der Erfindung wird der Ballon aus mehreren, mindestens 2, bevorzugt 3, einzelnen Segmenten zusammengesetzt, wobei die Fläche mindestens eines Segmentes von einer Rechteckfläche abweicht. Auf diese Weise lässt sich der Ballon derart zusammensetzten, dass sich im expandierten Zustand eine nicht zylindrische, gebogene oder gewundene Form ergibt.

Unter einem Segment wird im Rahmen der Erfindung ein Stück eines Ballons verstanden, welches mit mindestens zwei anderen Segmenten zusammen den vollständigen Ballon bildet. Ein Segment ist somit ein Teilstück der Oberfläche des Ballons beliebiger Fläche. Eine Kugel, wie beispielsweise ein Fußball, lässt sich aus einer Vielzahl von Sechsecken und Fünfecken zusammensetzen. Im Rahmen dieser Anmeldung würde man diese Sechsecke und Fünfecke als Segmente mit einer sechseckigen bzw. fünfeckigen nicht rechteckigen Fläche verstehen. Die jeweiligen Kontaktbereiche des Ballons mit dem Schaft des Ballonkatheters werden im Rahmen der Anmeldung nicht als Segmente verstanden. Die Kontaktbereiche oder Kontaktstellen bilden ein eher kegelförmiges Segment, das nicht exakt definierbar ist, und werden daher im Rahmen dieser Anmeldung und im Sinne dieser Ausgestaltung der Erfindung nicht als Segment gesehen. Im Rahmen dieser Ausgestaltung ist der Hauptteil des Ballons, d.h. die hauptsächliche Form des Ballons ohne den Kontaktbereich des Ballons mit dem Schaft des Ballonkatheters aus mindestens zwei, bevorzugt drei, einzelnen Segmenten zusammengesetzt.

Ein Segment ist im Rahmen der Erfindung keine gedankliche Teilung des Ballons in einzelne Abschnitte. Ein Ballonkatheter dieser Ausgestaltung der Erfindung weist einen Ballon auf, der aus mehreren Segmenten oder Einzelteilen zusammengesetzt ist. Die einzelnen Segmente werden dabei derart miteinander verbunden, dass der entstehende Ballon in seinem bestimmungsgemäßen Zustand mit einem Fluid druckdicht beaufschlagt werden kann.

In dieser Ausgestaltung der Erfindung hat mindestens ein Segment des Ballons eine nicht rechteckige Fläche. Ein Segment mit einer rechteckigen Grundfläche entspricht einem Zylindermantel und würde entsprechend dreidimensional zusammengesetzt eine zylindrische Form ergeben. Durch die Abweichung von der Form eines Rechteckes wird eine dreidimensionale Form erreicht, die sich von der Form eines Zylinders unterscheidet. Die Biegung des Ballons wird durch die Orientierung der Segmentkanten eingestellt

Bevorzugt sind die einzelnen Segmente an ihren Stoßkanten miteinander verschweißt oder verklebt, wobei die Stoßkanten zumindest leicht überlappen, um ein Verschweißen bzw. Verkleben zu gewährleisten. Durch das Verschweißen oder Verkleben der einzelnen Segmente wird der gesamte Ballon gebildet. Die einzelnen Segmente sind dadurch druckfest zu einem expandierbaren Ballon verbunden.

Ein Ballonkatheter dieser Ausgestaltung der Erfindung lässt sich daher dadurch herstellen, dass einzelne Segmente aus Ballonmaterial zugeschnitten werden. Die einzelnen Segmente werden dann derart miteinander verschweißt oder verklebt, dass die gewünschte dreidimensionale Form des Ballons entsteht. Die Verbindungsbereiche des Ballons mit dem Schaft des Ballonkatheters können in dieser Ausgestaltung der Erfindung auf verschiedene Weise realisiert werden.

Zum einen kann wie im Stand der Technik Ballonmaterial erwärmt und unter Druck gedehnt werden. Dies führt im Stand der Technik zu einem zylindrischen Ballon. Dessen Randbereiche werden abgeschnitten und mit den Segmenten dieser Ausführungsform der Erfindung verklebt oder verschweißt. Die Randbereiche dienen dann der Verbindung eines Ballons dieser Ausführungsform der Erfindung mit dem Schaft des Ballonkatheters. Alternativ können auch speziell Randbereiche aus Ballonmaterial zugeschnitten werden und mit den Segmenten dieser Ausführungsform der Erfindung mittels verschweißen oder verkleben verbunden werden.

Vorteilhafterweise weist der Ballon im expandierten Zustand eine Form auf, die aus mindestens zwei zylinderförmigen Abschnitten besteht, wobei die Hauptachsen von mindestens zwei zylinderförmigen Abschnitten einen Winkel mehr als 0° und weniger als 180°, bevorzugt mehr als 0° und weniger als 90°, aufweisen.

Unter der Hauptachse eines zylinderförmigen Abschnittes wird in dieser Ausgestaltung der Erfindung die axiale Symmetrieachse des zylinderförmigen Abschnittes verstanden, d.h. die Achse vom Mittelpunkt der fiktiven kreisförmigen Grundfläche zum Mittelpunkt der fiktiven kreisförmigen Deckfläche des zylindrischen Abschnittes.

Dies ist die einfachste und zweckmäßigste Realisierung dieser Ausführungsform der Erfindung. Der Ballon besteht zweckmäßigerweise aus drei verschiedenen Segmenten. Zwei Segmente mit rechteckiger Fläche werden über ein drittes Segment mit nicht rechteckiger Fläche verbunden. Die beiden Segmente mit rechteckiger Fläche bilden zylinderförmige Abschnitte. Die rechteckige Fläche der Segmente ist die Mantelfläche des Zylinders dieses Abschnittes. Die Kanten dieser beiden Segmente werden mit den Kanten des dritten Segmentes verbunden, welches eine Fläche aufweist, die kein Rechteck ist. Dadurch wird erreicht, dass die Hauptachsen der beiden zylindrischen Abschnitte einen Winkel aufweisen, welcher größer als 0° und kleiner als 180° ist. D.h. im expandierten Zustand des Ballons dieser Ausgestaltung werden zwei Zylinder über ein nicht zylindrisches Segment verbunden, wodurch sich automatisch eine Krümmung ergibt. Die beiden Hauptachsen der zylindrischen Abschnitte sind nicht mehr in einer Linie, der Ballon insgesamt ist im expandierten Zustand gekrümmt bzw. gebogen.

Die Krümmung dieser Ausgestaltung der Erfindung entspricht dabei der Krümmung und dem natürlichen Zustand des Körpergefäßes, in welches der Ballon zur Expansion, und insbesondere zum Einbringen eines Stents, eingebracht wird. Die Krümmung des expandierten Ballons kann dabei ebenso wie der Winkel zwischen den zylindrischen Segmenten des Ballons vorteilhafterweise beliebig variiert werden, wobei der Behandler entsprechend dem zu behandelnden Körpergefäß zwischen verschiedenen derartigen Ballonen auswählt.

Je nach Zuschnitt des dritten Segmentes zwischen den zylindrischen Segmenten lässt sich der Winkel zwischen den Hauptachsen der zylindrischen (und somit die Krümmung des expandierten Ballons) Segmente mehr oder weniger groß gestalten. Der Radius der Krümmung ist in dieser Ausgestaltung der Erfindung durch Größe, Zahl und Winkel der einzelnen Segmente zueinander steuerbar. Wird mehr als ein drittes Segment zwischen die beiden zylindrischen Segmente eingefügt, lässt sich eine Biegung feiner/ genauer beschreiben. Je mehr und je feiner segmentiert, umso weniger scharf ist der Ballon im expandierten Zustand gebogen. Zusätzlich lässt sich vorteilhafterweise über die Zahl und den Zuschnitt der Segmente jede beliebige dreidimensionale Form des Ballons im expandierten Zustand erreichen.

Zweckmäßigerweise werden die einzelnen Segmente nicht nur miteinander sondern auch mit sich selbst derart verbunden, dass ein Hohlkörper entsteht. Dabei wird bevorzugt ein Segment mit sich selbst derart zu einem Hohlkörper verbunden, dass die Verbindung in einer Linie mit der Verbindung des benachbarten Segmentes mit sich selbst liegt. Diese Verbindungslinie ist bevorzugt der innere Radius des gebogenen Ballons im expandierten Zustand.

Diese Ausgestaltung der Erfindung beschreibt somit einen Ballonkatheter mit einem Ballon, der im expandierten Zustand eine beliebige gewundene oder gebogene dreidimensionale Form einnimmt. Bei der Behandlung einer Stenose in einem Körpergefäß, insbesondere beim Einbringen eines Stents in dieses Körpergefäß, wird ein Ballonkatheter nach dieser Ausgestaltung der Erfindung vorteilhafterweise derart ausgewählt, dass die gekrümmte oder gebogene dreidimensionale Form des Ballons im expandierten Zustand mit der gekrümmten oder gebogenen dreidimensionalen Form des Körpergefäßes übereinstimmt. Der Behandler wählt dabei den Ballonkatheter dieser Ausgestaltung der Erfindung entsprechend aus mehreren derartigen Ausgestaltungen der Erfindungen mit Ballonen verschiedenster gewundener oder gebogener Form und Größe aus. Die Begradigung des Körpergefäßes und die damit einhergehenden Probleme des Standes der Technik werden in dieser Ausgestaltung der Erfindung vermieden.

In einer anderen Ausgestaltung der Erfindung ist mindestens ein Draht mit dem Material des Ballons in geeigneter Weise derart verbunden, dass sich die Nachgiebigkeit des Ballons im nicht expandierten Zustand in mindestens einem Abschnitt entlang einer ersten Kurve auf dem Ballon von der Nachgiebigkeit entlang einer zweiten Kurve auf dem Ballon unterscheidet.

Unter der Nachgiebigkeit des Ballons wird im Rahmen dieser Anmeldung und dieser Ausgestaltung der Erfindung die Reaktion des Ballons bei Druckänderung/Druckerhöhung bezeichnet. Der Ballon wird mittels eines Fluids im Balloninneren unter Druck gesetzt und dadurch expandiert. Die Nachgiebigkeit beschreibt, wie stark die Expansion bei vorgegebenem Druck ist. Ein Ballon höherer Nachgiebigkeit dehnt sich bei gleichem Druck stärker aus als ein Ballon mit geringerer Nachgiebigkeit. In der englischen Literatur wird die Nachgiebigkeit auch als Compliance bezeichnet. Wie stark ein Ballon in radialer Richtung nachgiebig ist, hat wenig direkten Einfluss, wie gut sich ein Ballon an das Gefäß anschmiegt. Dies hängt eher von der Biegesteifigkeit im expandierten Zustand ab.

Unter einer Kurve wird im Rahmen der Erfindung eine stetige Punktfolge im geometrischen Raum verstanden. Im einfachsten Fall ist eine Kurve eine Gerade auf dem Ballonmaterial.

In dieser Ausgestaltung der Erfindung wird eine nicht-zylindrische Form des Ballons des erfindungsgemäßen Ballonkatheters durch eine gezielte, lokale Änderung der Nachgiebigkeit des Ballons in einem vorgegebenen Abschnitt entlang einer vorgegebenen Linie. Durch die Verbindung eines Drahtes mit dem Ballonmaterial wird ein gezielter Eingriff in die Nachgiebigkeit des Ballons vorgenommen. Im einfachsten Fall wird die Nachgiebigkeit des Ballons entlang der Verbindungslinie von Ballon und Draht verringert.

Bevorzugt ist ein Draht entlang einer Kurve mit dem Material des Ballons, bevorzugt im Inneren des Ballons, verschweißt oder verklebt oder in das Ballonmaterial integriert.

Diese Ausgestaltung der Erfindung umfasst verschiedenste Ausführungsformen. Der Draht kann zweckmäßigerweise die gleiche Länge wie der Ballon von proximal nach distal aufweisen. Alternativ sind aber auch sowohl kürzere als auch längere Drähte möglich. Ebenso werden von dieser Ausgestaltung der Erfindung verschiedene Formen von Drähten (runde Drähte, Profildrähte oder flache Streifen ebenso wie gerade Drähte, Spiraldrähte oder ähnliches) und Materialien (metallischer oder nicht metallischer Federstahl, Angelsehne) umfasst. Die Wahl von Form, Material und Länge des Drahtes hängt dabei von der gewünschten Form des Ballons im expandierten Zustand ebenso wie von der bevorzugten Fertigung ab. Der Begriff Draht ist daher im Rahmen dieser Anmeldung nicht auf metallische Drähte beschränkt, sondern beschreibt generell eine drahtähnliche Sehne.

So kann in dieser Ausgestaltung der Erfindung beispielsweise ein Ballon mit starker Krümmung durch die Verbindung mit einem kurzen, sehr steifen Draht erreicht werden. Durch eine enge Verknüpfung eines derartigen Drahtes mit dem Ballonmaterial, beispielsweise durch Verschweißen, wird die Nachgiebigkeit des Ballons entlang des Drahtes drastisch verringert. Dadurch dehnt sich der Ballon im expandierten Zustand entlang des Drahtes deutlich weniger aus, wodurch eine gekrümmte dreidimensionale Form des Ballons erreicht wird. Durch den Einsatz mehrerer verschiedener derartiger Drähte lässt sich zweckmäßigerweise eine beliebige dreidimensionale Form des Ballons im expandierten Zustand erreichen. Ähnlich der ersten Ausgestaltung der Erfindung kann so durch den Behandler ein Ballonkatheter mit einem Ballon vorgegebener dreidimensionaler Form im expandierten Zustand derart ausgewählt werden, dass die natürliche Form des Körpergefäßes nachgebildet wird. Entsprechend wird bei Expansion des Ballons, insbesondere beim Einbringen eines Stents in das Körpergefäß, das Körpergefäß nicht begradigt, wodurch die geschilderten Nachteile des Standes der Technik vermieden werden.

In einer bevorzugten Ausführungsform dieser Ausgestaltung ist die Kurve im nicht expandierten Zustand des Ballons eine Gerade, die sich bevorzugt vom proximalen bis zum distalen Ende des Ballons erstreckt.

In dieser Ausführungsform der Erfindung wird ein gerader Draht über die gesamte Länge des Ballons von proximal nach distal mit dem Ballon verbunden. Bevorzugt wird der Draht im Balloninneren an mehreren Punkten, bevorzugt entlang der gesamten Länge, mit dem Ballonmaterial verschweißt oder verklebt. Derart wird in dieser Ausgestaltung der Erfindung die Steifigkeit des Ballons entlang einer Gerade von proximal nach distal erhöht. Die Nachgiebigkeit des restlichen Ballons bleibt unbeeinflusst, lediglich entlang des Drahtes von proximal nach distal ist der Ballon steifer. Entsprechend dehnt sich der Ballon bei Expansion durch die Beaufschlagung mit einem Fluid entlang dieser Linie deutlich weniger aus. Dadurch nimmt der Ballon im expandierten Zustand eine gebogene dreidimensionale Form, ähnlich einer Banane, ein. Der Draht kann dabei im Balloninneren Punktweise angeheftet oder komplett verschweißt bzw. verklebt sein. Alternativ kann der Draht direkt in das Ballonmaterial integriert sein. Eine derartige Ausführungsform kann beispielsweise dadurch hergestellt werden, dass ein Draht auf dem inneren Ballon platziert und fixiert wird und diese Konstruktion in einen äußeren Ballon mit entsprechend großen proximalen Hals eingeführt wird.

In einer besonders bevorzugten Ausführungsform dieser Ausgestaltung ist der gerade Draht(bzw. besonders zugfeste Innenschaft) nur mittig z.B. am proximalen und distalen Ballonhals mit dem Ballonmaterial verknüpft. In dieser Ausgestaltung der Erfindung wird die durch den Balloninnendruck erzeugte axiale Spannung in der Ballonmembran zum Teil oder ganz durch den Draht übernommen. Die Ballonmembran ist damit nicht über dem gesamten Umfang in axialer Richtung gespannt. Es ergibt sich automatisch eine gebogene dreidimensionale Form ähnlich einer Banane. Auf der Außenseite der Krümmung ist die Ballonmembran gespannt, auf der Innenseite legt sie sich in Falten. Diese besonders bevorzugte Form passt sich der dreidimensionalen Windung des Körpergefäßes an, ist also "conformable".

Entsprechend wird automatisch die Nachgiebigkeit des Ballons entlang der Innenseite der Krümmung verringert und der Ballon folgt im expandierten Zustand der natürlichen Krümmung des Körpergefäßes. Die geschilderten Nachteile des Standes der Technik werden so optimal vermieden.

In einer anderen bevorzugten Ausführungsform dieser Ausgestaltung ist die Kurve eine Helix, wobei sich entlang einer ersten geraden Achse auf dem Ballon von proximal nach distal weniger Ballonmaterial zwischen zwei benachbarten Schnittpunkten der ersten Kurve und der Helix befindet als zwischen den benachbarten Schnittpunkten einer zweiten Kurve auf dem Ballon von proximal nach distal und der Helix, wobei erste und zweite Kurve im nicht expandierten Zustand des Ballons Geraden sind.

In einer besonders bevorzugten Form dieser Ausgestaltung erstreckt sich die Helix sowie die erste als auch die zweite Achse vom proximalen bis zum distalen Ende des Ballons, wobei sich bevorzugt die erste und die zweite Achse auf gegenüberliegenden Seiten des Ballons befinden. In dieser Ausführungsform der Erfindungen werden die Windungen einer Helix mit dem Ballonmaterial verbunden, wobei die Helix im einfachsten Fall die gleiche Länge wie der Ballon aufweist. Dabei werden die Helixwindungen entlang zweier Geraden mit dem Ballonmaterial verbunden. Diese beiden Geraden liegen sich bevorzugt gegenüber, wobei sich entlang der einen Geraden mehr Ballonmaterial zwischen zwei benachbarten Helixwindungen befindet als entlang der gegenüberliegenden Geraden. D.h. entlang der einen Geraden wird das Ballonmaterial zwischen zwei benachbarten Verknüpfungen ähnlich einer Gardine gerafft, d.h. die Länge des Ballonmaterials zwischen 2 benachbarten Windungen des helixförmigen Drahtes ist größer als der Abstand zwischen den beiden Windungen. Entsprechend befindet sich so mehr Ballonmaterial zwischen den benachbarten Helixwindungen als entlang der gegenüberliegenden Gerade. Wird der Ballon durch Beaufschlagung mit dem Fluid expandiert, kann sich der Ballon entlang der Geraden mit Materialüberschuss mehr ausdehnen als entlang der gegenüberliegenden Geraden. Die gegenüberliegende Gerade bildet somit eine Kurve mit höherer Steifigkeit, wodurch sich ähnlich der vorangegangenen Ausführungsform eine gekrümmte dreidimensionale Form des Ballons ergibt.

Für die Verbindung des helixförmigen Drahtes mit dem Ballonmaterial und die Materialien gelten die Ausführungen der vorangegangenen Abschnitte analog.

In dieser Ausgestaltung der Erfindung können beliebige gebogene dreidimensionale Formen des Ballons im expandierten Zustand realisiert werden, je nach dem wie die erste und zweite Kurve gewählt werden. Beide können sich über die ganze Länge des Ballons von proximal nach distal oder kürzer erstrecken. Ebenso ist es bei einigen Anwendungsfällen zweckmäßig, wenn sich beide Kurven kreuzen, während sie in anderen Anwendungsfällen auch in einer Linie angeordnet sein können. Durch die Verringerung der Nachgiebigkeit des Ballons entlang der beliebigen ersten Kurve durch die Reduktion des Ballonmaterials zwischen den Helixwindungen wird an diesen Stellen immer eine Krümmung erreicht. Entsprechend sind auch Ausgestaltungen analog zum vorangegangen Abschnitt mit mehr als 2 Kurven möglich. Ebenso kann der Ballon zweckmäßigerweise Abschnitte aufweisen, die unterschiedlich mit Kurven und unterschiedlichen helixförmigen Drähten versehen sind.

In einer weiteren Ausgestaltung der Erfindung befinden sich im Inneren des Ballons mindestens 2 Drähte, die derart miteinander verbunden sind, dass sich die Nachgiebigkeit des Ballons im nicht expandierten Zustand in mindestens einem Abschnitt entlang einer ersten Kurve auf dem Ballon von der Nachgiebigkeit entlang einer zweiten Kurve auf dem Ballon unterscheidet. Bevorzugt ist dabei ein helixförmiger Draht mit einem ersten Draht an mindestens zwei benachbarten Windungen der Helix verbunden ist, wobei sich bevorzugt beide Drähte vom proximalen zum distalen Ende des Ballons erstrecken. Besonders bevorzugt ist der erste Draht gerade.

In einer ähnlichen Ausgestaltung der Erfindung ist ein helixförmiger Draht an mindestens zwei benachbarten Windungen der Helix mit einem ersten Draht und an mindestens zwei benachbarten Windungen der Helix mit einem zweiten Draht verbunden. Bevorzugt unterscheidet sich die Länge des ersten Drahtes zwischen zwei benachbarten Windungen der Helix von der Länge des zweiten Drahtes zwischen zwei benachbarten Windungen der Helix, wobei sich bevorzugt sowohl der helixförmige Draht als auch der erste und der zweite Draht vom proximalen bis zum distalen Ende des Ballons erstrecken. Besonders bevorzugt sind der erste und der zweite Draht gerade, insbesondere ist der erste Draht auf der dem zweiten Draht gegenüberliegenden Seite der Helix mit dem helixförmigen Draht verbunden. Zweckmäßigerweise erstrecken sich sowohl der helixförmige Draht als auch der erste und der zweite Draht über die gesamte Länge des Ballons von proximal nach distal.

Die letzten beiden geschilderten Ausgestaltungen ähneln der Ausgestaltung der unmittelbar vorangegangenen Absätze. In diesen Ausgestaltungen wird ein helixförmiger Draht als eine Art Gerüst in das Balloninnere eingebracht. Die Nachgiebigkeit des Ballons wird bewusst lokal durch das Einbringen von einem oder mehreren Drähten verbunden mit den Windungen des helixförmigen Drahtes manipuliert. Dadurch kann der Abstand bzw. die mögliche Entfernung in Abhängigkeit vom angelegten Druck der Helixwindungen des Drahtes und somit des Ballons manipuliert werden. In diesen Ausgestaltungen wird daher analog die Nachgiebigkeit lokal verändert und gekrümmte oder gebogene dreidimensionale Formen des Ballons des Ballonkatheters im expandierten Zustand erreicht. Daher kann auch in diesen Ausgestaltungen des erfindungsgemäßen Ballonkatheters die Begradigung des Körpergefäßes insbesondere beim Einbringen eines Stents in das Körpergefäß und die damit einhergehenden Nachteile des Standes der Technik vermieden werden.

In einer weiteren alternativen Ausgestaltung des erfindungsgemäßen Ballonkatheters, weist dieser im distalen Bereich einen inneren Schaft mit einem inneren Lumen und ein äußeres zweites Lumen auf, wobei das zweite Lumen eine Strömungsverbindung mit dem Balloninneren derart aufweist, dass der Ballon durch ein Fluid im zweiten Lumen expandiert werden kann und die Länge des Ballons von proximal nach distal größer als die Länge des Innenschafts vom proximalen Ende des Ballons zum distalen Ende des Ballons ist.

Bevorzugt ist die Länge des Ballons mit Durchmesser d von proximal nach distal L*(1-r/(r+d)) größer ist als die Länge L des Innenschafts vom proximalen Ende des Ballons zum distalen Ende des Ballons ist, um einem Krümmungsradius r des Körpergefäßes folgen zu können.

In dieser Ausgestaltung der Erfindung weist der Ballon eine größere Länge von proximal nach distal als der Innenschaft auf. Dies führt zu einer nicht zylindrischen Form im expandierten Zustand des Ballons. Der Ballon nach dieser Ausgestaltung der Erfindung passt sich dem gewundenen oder gebogenen dreidimensionalen Verlauf des Körpergefäßes an, in dem der Ballon expandiert wird. Dies wird durch den Materialüberschuss des Ballons, durch seine größere Länge, gewährleistet. Der Ballon ist länger bzw. es ist mehr Ballonmaterial vorhanden als eigentlich notwendig. Der kürzere Innenschaft folgt dem Verlauf des Körpergefäßes auf dem kürzesten Weg, d.h. er schneidet quasi die Krümmungen und Biegungen des Gefäßverlaufes. Durch die größere Länge kann der Ballon jedoch dem natürlichen gebogenen oder gewundenen dreidimensionalen Verlauf des Körpergefäßes folgen. Dadurch wird eine erzwungene Begradigung des Gefäßes wie bei übereinstimmender Länge von Innenschaft und expandierten Ballon entsprechend dem Stand der Technik mit den vorangehend geschilderten Nachteilen vermieden.

Bevorzugt weist der Ballonkatheter Mittel auf, mit denen die Größe des Längenunterschiedes zwischen Ballon und Innenschaft variabel eingestellt werden kann. Insbesondere ist das distale Ende des Ballons druckdicht und beweglich über den inneren Schaft. Ein derartiges Mittel wäre beispielsweise eine gleitende Dichtung.

Bei dem erfindungsgemäßen Ballonkatheter ist insbesondere ein Stent auf dem Ballon aufgebracht.

Die einzelnen Ausgestaltungen der Erfindung können beliebig miteinander kombiniert werden.

Die Anmeldung betrifft ferner ein System zum Einbringen eines Stents in ein Körpergefäß mit einem Ballonkatheter wie in den vorangegangenen Absätzen beschrieben.

Mit Hilfe des erfindungsgemäßen Ballonkatheters sowie dem erfindungsgemäßen System zum Einbringen eines Stents in ein Körpergefäß wird das Begradigen des Körpergefäßes durch die Expansion des Ballons und das Einbringen des Stents vermieden. Dadurch werden die natürlichen Strömungsverhältnisse des Körpergefäßes vor der Stenose viel besser wieder hergestellt/erhalten. Gleichzeitig werden die im Stand der Technik geschilderten Nachteile (Strömungstotstellen im Innenbereich bei eingesetzten Stent; Eindrücken des Stents in die Gefäßwand im Außenbereich des gekrümmten Körpergefäßes) vermieden und das Risiko einer Restenose entsprechend gesenkt.

Im Folgenden soll die Erfindung anhand der in den Figuren dargestellten Ausführungsbeispiele näher erläutert werden.

Es zeigen:
- Fig. 1: eine Körpergefäß mit einem Stent nach Einsetzen des Stents mit einem Ballonkatheter nach dem Stand der Technik,
- Fig. 2: eine Ausgestaltung des erfindungsgemäßen Ballonkatheters mit einem Ballon aus einzelnen Segmenten,
- Fig. 3: die dreidimensionale Darstellung des Ballons der Ausgestaltung mit drei Segmenten,
- Fig. 4: eine Ausgestaltung des erfindungsgemäßen Ballonkatheters mit einem Ballon mit einem Draht,
- Fig. 5: eine Ausgestaltung des erfindungsgemäßen Ballonkatheters mit einem Ballon mit einem helixförmig gewundenen Draht und einem ersten sowie einem zweiten geraden Draht und
- Fig. 6: eine Ausgestaltung des erfindungsgemäßen Ballonkatheters mit einem Ballon der eine größere Länge von proximal nach distal als der Innenschaft aufweist.

Figur 1 wurde bereits in der Beschreibung bei der Schilderung des Standes der Technik beschrieben.

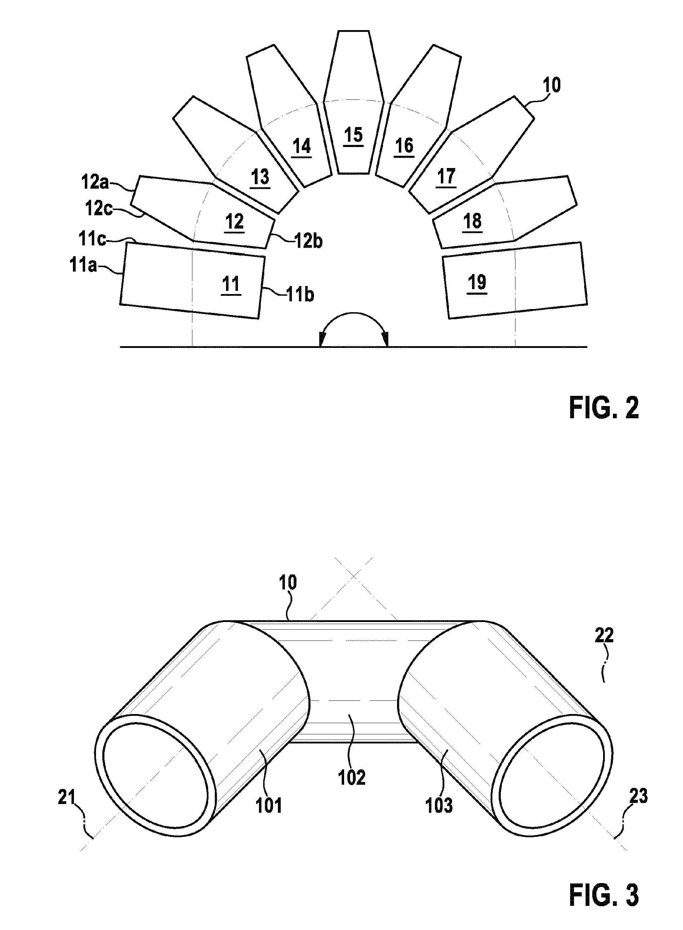

Figur 2 zeigt schematisch einen Ballon 10 der aus den einzelnen Segmenten 11 bis 19 besteht, wobei die Flächen der einzelnen Segmente dargestellt sind. Bei der in Figur 2 dargestellten Ausführungsform besteht der Ballon 10 aus neun einzelnen Teilen 11 bis 19. Dabei weisen nur die Segmente 11 und 19 eine rechteckige Fläche auf. Die übrigen Segmente 12 bis 18 weisen eine nicht rechteckige Fläche in Form eines lang gezogenen Sechseckes auf. Der eigentliche Ballon 10 wird aus den einzelnen Segmenten 11 bis 19 zusammengesetzt, was beispielhaft an den Segmenten 11 und 12 beschrieben wird.

Bei Segment 11 werden die beiden Seitenkanten 11a und 11b mit einander verbunden. Derart entsteht ein zylinderförmiger Hohlkörper. Bei Segment 12 werden ebenfalls die beiden Seitenkanten 12a und 12b miteinander verbunden, so dass ebenfalls ein Hohlkörper entsteht. Die beiden Segmente 11 und 12 werden anschließend an ihren Seitenkanten 11c und 12c miteinander verbunden. Durch die nicht rechteckige Fläche von Segment 12 weicht die Form der kombinierten Segmente 11 und 12 von der Zylinderform ab. Es entsteht ein Hohlkörper mit einer Biegung. In analoger Weise werden alle Segmente 11 bis 19 miteinander verbunden. Auf diese Weise lässt sich Ballon 10 aus den Einzelsegmenten 11 bis 19 formen, so dass der Ballon 10 in seinem expandierten Zustand eine gebogene dreidimensionale Form in Form eines U aufweist. Wie angedeutet lässt sich dabei der Radius der Biegung durch die Zahl und Dimension der einzelnen Segmente 12 bis 18 vorgeben.

Figur 3 zeigt schematisch als dreidimensionale Darstellung einer Ausgestaltung eines erfindungsgemäßen Ballonkatheters mit der einfachste Form eines Ballons 10 aus einzelnen Segmenten. Der Ballon 10 besteht aus drei Segmenten 101, 102 und 103 und ist in seinem expandierten Zustand dargestellt. Der Ballon 10 besteht aus zwei Segmenten 101 und 103 mit rechteckiger Fläche und einem Segment 102 mit einer nicht rechteckigen Fläche. Durch die nicht rechteckige Fläche des mittleren Segments 102 wird eine gebogene dreidimensionale Form des Ballons 10 in seinem expandierten Zustand erreicht. Die Hauptachsen 21, 22 und 23 der drei zylindrischen Abschnitte 101, 102 und 103 weisen dabei jeweils einen Winkel von über 0° und weniger als 90° zueinander auf.

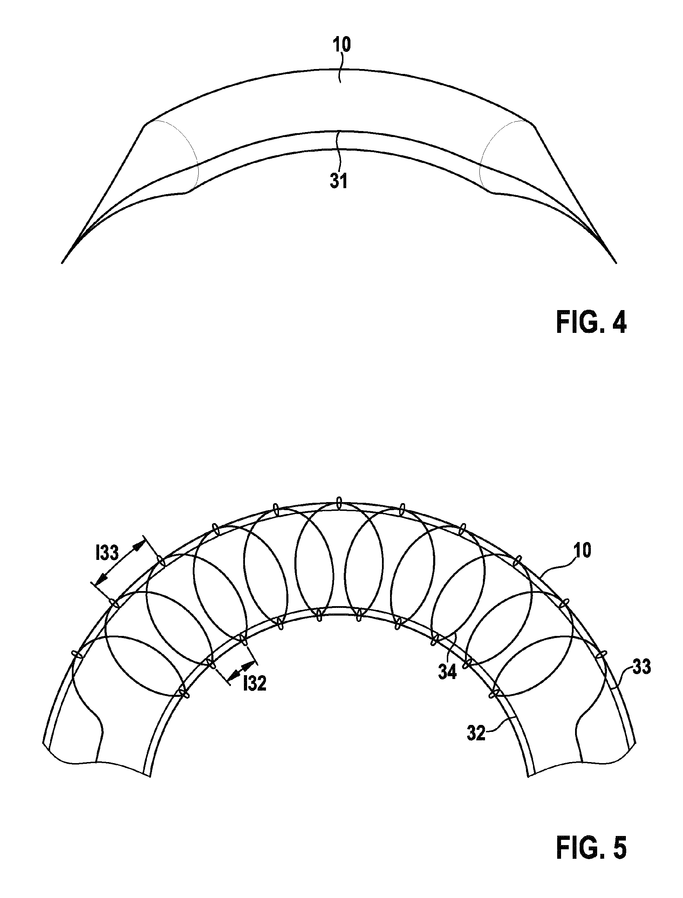

Figur 4 zeigt schematisch den Ballon 10 einer Ausgestaltung eines erfindungsgemäßen Ballonkatheters, wobei der Ballon 10 einen Draht 31 aufweist. Der Draht 31 erstreckt sich in dieser Ausgestaltung der Erfindung als Gerade über die gesamte Länge des Ballons 10 von proximal nach distal. Der Draht 31 ist dabei mit dem Ballonmaterial des Ballons 10 verbunden (verschweißt, verklebt oder in das Ballonmaterial eingebracht). Durch den Draht 31 wird die Steifigkeit des Ballons 10 lokal verändert. Konkret wird die Steifigkeit entlang des Drahtes 31 stark erhöht. Dadurch kann sich der Ballon 10 bei Druckbeaufschlagung durch das Fluid entlang des Drahtes deutlich weniger ausdehnen, so dass der Ballon 10 im expandierten Zustand die gebogene dreidimensionale Form einer Banane aufweist.

Figur 5 zeigt schematisch den Ballon 10 einer Ausgestaltung eines erfindungsgemäßen Ballonkatheters, wobei der Ballon 10 drei Drähte 32, 33 und 34 aufweist. Dabei dient der helixförmige Draht 34 als Gerüst für den Ballon 10. In dieser Ausgestaltung der Erfindung wird die Steifigkeit des Ballons 10 lokal durch die beiden Drähte 32 und 33 verändert. In dieser Ausgestaltung der Erfindung sind die beiden Drähte 32 und 33 fest mit den Windungen des helixförmigen Drahtes 34 verbunden. Die Verbindung erfolgt hier durch ein Durchfädeln der Drähte. Entscheidend ist in dieser Ausgestaltung die Länge 132 und 133 zwischen zwei benachbarten Windungen des helixförmigen Drahtes 34. In dieser Ausgestaltung der Erfindung ist die Länge 132 deutlich kleiner als 133, wodurch die Steifigkeit des Ballons 10 entlang des Drahtes 32 deutlich erhöht ist. Entsprechend dehnt sich der Ballon 10 bei Beaufschlagung mit einem Fluid entlang des Drahtes 32 weniger aus und nimmt ähnlich der Ausgestaltung der Erfindung nach Figur 4 eine gebogene dreidimensionale Form ähnlich einer Banane an.

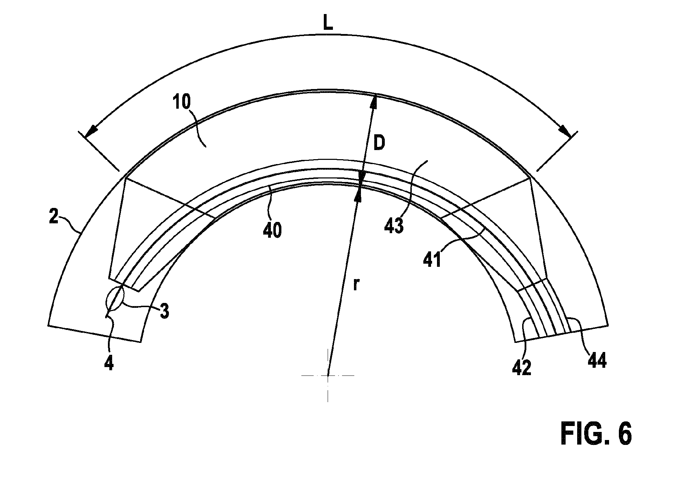

Figur 6 zeigt schematisch eine Ausgestaltung der Erfindung, bei dem die Länge des Innenschafts 40 kleiner als die Länge des Ballons 10 ist. Der Ballonkatheter ist hier im Körpergefäß 2 dargestellt. Der Ballonkatheter weist einen Innenschaft 40 mit einem inneren Lumen 41 sowie einen Außenschaft 44 mit einem äußeren Lumen 42 auf. Das äußere Lumen 42 ist zwischen Innenschaft und Außenschaft und in Strömungsverbindung mit dem Balloninneren 43. Über das äußere Lumen 42 wird der Ballon 10 mit einem Fluid beaufschlagt und expandiert. Im inneren Lumen 41 befindet sich der Führungsdraht 4. Am distalen Ende des Ballonkatheters befindet sich eine Spitze 3 aus weichem röntgensichtbaren Material.

In dieser Ausgestaltung der Erfindung ist die Länge des Innenschafts 41 vom proximalen zum distalen Ende des Ballons 10 kleiner als die Ballonlänge. Dadurch wird in dieser Ausgestaltung der Erfindung eine dreidimensional gekrümmte Form des Ballons 10 in seinem expandierten Zustand sichergestellt. Der Innenschaft 40 folgt dem dreidimensionalen Verlauf des Gefäßes 2 auf direktem Weg. Dadurch, dass die Ballonlänge größer ist als die Länge des Innenschafts vom proximalen zum distalen Ende des Ballons, wird sichergestellt, dass der Ballon sich im expandierten Zustand dem dreidimensionalen Verlauf des Körpergefäßes 2 anpasst.

Bei der in Figur 6 gezeigten Ausgestaltung der Erfindung ist die Länge des Ballons 10 mit Durchmesser D von proximal nach distal L*(1-r/(r+D)) größer als die Länge L des Innenschafts 40 vom proximalen Ende des Ballons 10 zum distalen Ende des Ballons 10, um einem Krümmungsradius r des Körpergefäßes 2 folgen zu können. Die Länge L des Ballons 10 wird dabei ohne die konischen Endbereiche bestimmt, der Durchmesser D wird im expandierten Zustand bestimmt.

## Patentansprüche

1. Ballonkatheter mit einem expandierbaren Ballon (10) zur Behandlung von Stenosen in Körpergefäßen (2), **dadurch gekennzeichnet, dass** der Ballon (10) derart ausgeformt ist, dass er im expandierten Zustand eine Form einnimmt, die von der Form eines Zylinders abweicht, insbesondere der Ballon im expandierten Zustand eine gebogene oder gewundene dreidimensionale Form aufweist.

2. Ballonkatheter nach Anspruch 1, **dadurch gekennzeichnet, dass** der Ballon (10) aus mindestens zwei, bevorzugt drei, einzelnen Segmenten (11, 12, 13, 14, 15, 16, 17, 18, 19, 101, 102, 103) zusammengesetzt ist, wobei mindestens ein Segment (12, 13, 14, 15, 16, 17, 18, 102) eine Fläche aufweist, die kein Rechteck ist.

3. Ballonkatheter nach Anspruch 2, **dadurch gekennzeichnet, dass** die einzelnen Segmente (11, 12, 13, 14, 15, 16, 17, 18, 19, 101, 102, 103) an ihren Stoßkanten miteinander verschweißt oder verklebt sind.

4. Ballonkatheter nach Anspruch 2 oder 3, **dadurch gekennzeichnet**, der Ballon (10) im expandierten Zustand eine Form aufweist, die aus mindestens zwei zylinderförmigen Abschnitten (101, 102, 103) besteht, wobei die Hauptachsen (21, 22, 23) von mindestens zwei zylinderförmigen Abschnitten (101, 102, 103) einen Winkel mehr als 0° und weniger als 180°, bevorzugt mehr als 0° und weniger als 90°, aufweisen.

5. Ballonkatheter nach Anspruch 1, dass mindestens ein Draht (31, 32, 33, 34)mit dem Material des Ballons (10) in geeigneter Weise derart verbunden ist, dass sich die Steifigkeit des Ballons (10) im nicht expandierten Zustand in mindestens einem Abschnitt entlang einer ersten Linie auf dem Ballon (10) von der Steifigkeit entlang einer zweiten Linie auf dem Ballon (10) unterscheidet.

6. Ballonkatheter nach Anspruch 5, **dadurch gekennzeichnet, dass** der/die Draht/Drähte (31, 32, 33, 34) entlang einer Kurve mit dem Material des Ballons (10), bevorzugt im Inneren des Ballons, verschweißt oder verklebt ist oder in das Ballonmaterial integriert ist.

7. Ballonkatheter nach Anspruch 6, **dadurch gekennzeichnet, dass** die Kurve im nicht expandierten Zustand des Ballons (10) eine Gerade ist und sich bevorzugt vom proximalen bis zum distalen Ende des Ballons erstreckt.

8. Ballonkatheter nach Anspruch 5 oder 6, **dadurch gekennzeichnet, dass** die Kurve eine Helix ist, wobei sich entlang einer ersten geraden Achse auf dem Ballon (10) von proximal nach distal weniger Ballonmaterial zwischen zwei benachbarten Schnittpunkten der ersten Kurve und der Helix befindet als zwischen den benachbarten Schnittpunkten einer zweiten Kurve auf dem Ballon von proximal nach distal und der Helix, wobei erste und zweite Kurve im nicht expandierten Zustand des Ballons Geraden sind.

9. Ballonkatheter nach Anspruch 8, **dadurch gekennzeichnet, dass** sich die Helix sowie die erste als auch die zweite Kurve vom proximalen bis zum distalen Ende des Ballons (10) erstreckt, wobei sich bevorzugt die erste und die zweite Kurve auf gegenüberliegenden Seiten des Ballons (10) befinden.

10. Ballonkatheter nach Anspruch 1, **dadurch gekennzeichnet, dass** sich im Inneren des Ballons mindestens zwei Drähte befinden, die derart miteinander verbunden sind, dass sich die Nachgiebigkeit des Ballons im nicht expandierten Zustand in mindestens einem Abschnitt entlang einer ersten Linie auf dem Ballon von der Nachgiebigkeit entlang einer zweiten Linie auf dem Ballon unterscheidet.

11. Ballonkatheter nach Anspruch 10, **dadurch gekennzeichnet, dass** ein helixförmiger Draht (34) mit einem Draht (31, 32, 33) an mindestens zwei benachbarten Windungen der Helix verbunden ist, wobei sich bevorzugt beide Drähte vom proximalen zum distalen Ende des Ballons (10) erstrecken.

12. Ballonkatheter nach Anspruch 10, **dadurch gekennzeichnet, dass** ein helixförmiger Draht (34) an mindestens zwei benachbarten Windungen der Helix mit einem ersten Draht (32) und an mindestens zwei benachbarten Windungen der Helix mit einem zweiten Draht (33) verbunden ist.

13. Ballonkatheter nach Anspruch 12, **dadurch gekennzeichnet, dass** sich die Länge (132) des ersten Drahtes (32) zwischen zwei benachbarten Windungen der Helix (34) von der Länge (133) des zweiten Drahtes (33) zwischen zwei benachbarten Windungen der Helix (34) unterscheidet, wobei sich bevorzugt sowohl der helixförmige Draht (34) als auch der erste und der zweite Draht (32, 33) vom proximalen bis zum distalen Ende des Ballons (10) erstrecken.

14. Ballonkatheter nach Anspruch 1, welcher im distalen Bereich einen inneren Schaft (40) mit einem inneren Lumen (41) und ein äußeres zweites Lumen (42) aufweist, wobei das zweite Lumen (42) eine Strömungsverbindung mit dem Balloninneren (43) derart aufweist, dass der Ballon (10) durch ein Fluid im zweiten Lumen (42) expandiert werden kann , **dadurch gekennzeichnet, dass** die Länge des Ballons (10) von proximal nach distal größer als die Länge des Innenschafts (40) vom proximalen Ende des Ballons (10) zum distalen Ende des Ballons (10) ist.

15. Ballonkatheter nach Anspruch 14 **dadurch gekennzeichnet, dass** die Länge des Ballons mit Durchmesser d (10) von proximal nach distal L*(1-r/(r+d)) größer ist als die Länge L des Innenschafts (40) vom proximalen Ende des Ballons (10) zum distalen Ende des Ballons (10) ist, um einem Krümmungsradius r des Körpergefäßes folgen zu können.

16. Ballonkatheter nach Anspruch 14 oder 15, **dadurch gekennzeichnet, dass** der Ballonkatheter Mittel aufweist, mit denen die Größe des Längenunterschiedes zwischen Ballon (10) und Innenschaft (40) variabel eingestellt werden kann, insbesondere ist das distale Ende des Ballons (10) druckdicht und beweglich über den inneren Schaft.

17. Ballonkatheter nach einem der vorangegangenen Ansprüche, **dadurch gekennzeichnet, dass** ein Stent (1) auf dem Ballon (10) aufgebracht ist.

18. System zum Einbringen eines Stents (1) in ein Körpergefäß (2) mit einem Ballonkatheter nach einem der vorangegangenen Ansprüche.
